# EUROPÄISCHE PATENTSCHRIFT

(11) **EP 1 206 459 B1**
(45) Veröffentlichungstag und Bekanntmachung des Hinweises auf die Patenterteilung: **09.02.2005**
(21) Anmeldenummer: 00958331.1
(22) Anmeldetag: 31.07.2000
(51) Int. Cl.: C07D 301/12

(54) **VERFAHREN ZUR UMSETZUNG EINER ORGANISCHEN VERBINDUNG MIT EINEM HYDROPEROXID**
METHOD FOR REACTING AN ORGANIC COMPOUND WITH A HYDROPEROXIDE
PROCEDE POUR FAIRE REAGIR UN COMPOSE ORGANIQUE AVEC UN HYDROPEROXYDE

(30) Priorität: 04.08.1999 DE 19936547
(43) Veröffentlichungstag der Anmeldung: 22.05.2002
(73) Patentinhaber: BASF AKTIENGESELLSCHAFT, 67056 Ludwigshafen (DE)
(72) Erfinder: TELES, Joaquim, Henrique, D-67122 Altrip (DE); REHFINGER, Alwin, D-67112 Mutterstadt (DE); BASSLER, Peter, D-68519 Viernheim (DE); RIEBER, Norbert, D-68259 Mannheim (DE); WENZEL, Anne, D-76344 Eggenstein-Leopoldshafen (DE); WALCH, Andreas, D-74193 Schwaigern (DE); HARDER, Wolfgang, D-69469 Weinheim (DE)
(74) Vertreter: Isenbruck, Günter, Dr.
(86) Internationale Anmeldenummer: PCT/EP2000/007383
(87) Internationale Veröffentlichungsnummer: WO 2001/010855

(56) Entgegenhaltungen:
- EP-A- 0 230 949
- EP-A- 0 434 546
- EP-A- 0 712 852
- EP-A- 0 757 043
- EP-A- 0 930 308
- WO-A-99/01445
- CLERICI M G ET AL: "EPOXIDATION OF LOWER OLEFINS WITH HYDROGEN PEROXIDE AND TITANIUM SILICALITE" JOURNAL OF CATALYSIS,US,ACADEMIC PRESS, DULUTH, MN, Bd. 140, Nr. 1, 1. März 1993 (1993-03-01), Seiten 71-83, XP000562771 ISSN: 0021-9517 in der Anmeldung erwähnt

## Beschreibung

Die vorliegende Erfindung betrifft ein Verfahren zur Umsetzung einer organischen Verbindung mit einem Hydroperoxid unter Verwendung eines heterogenen Katalysators, wobei während der Umsetzung der organischen Verbindung sowohl der pH-Wert als auch die Temperatur des Reaktionsmediums verändert werden. In einer weiteren Ausführungsform betrifft die vorliegende Erfindung auch ein Verfahren, in dem zusätzlich zu pH-Wert und Temperatur des Reaktionsmediums auch der Druck verändert wird, unter dem die Umsetzung abläuft.

Bei Umsetzungen von organischen Verbindungen mit einem Hydroperoxid, die unter Verwendung eines heterogenen Katalysators durchgeführt werden, nimmt in der Regel die Aktivität des heterogenen Katalysators bei längerem Einsatz ab.

Um den Katalysator wiederverwenden zu können, ist es in diesen Fällen erforderlich, den Katalysator aus den Reaktoren, in denen die Umsetzungen durchgeführt werden, auszubauen und außerhalb der Reaktoren zu regenerieren. Bei heterogenen Titansilikalitkatalysatoren, die beispielsweise zur Umsetzung von Olefinen mit Hydroperoxidlösungen eingesetzt werden, werden solche Regenerierungsverfahren beispielsweise durch Calcinieren des Katalysators bei höherer Temperatur durchgeführt, wie es beispielsweise in J. Catal. 129 (1991) 159 - 166 beschrieben ist. Eine andere Möglichkeit, die ebenfalls in dieser Schrift angegeben ist, besteht darin, den Katalysator mit geeigneten Lösungsmitteln zu waschen. Da die Regenerierung nach diesen Verfahren nur nach Ausbau des Katalysators erfolgen kann, sind diese Verfahren aufwendig und daher verfahrensökonomisch unerwünscht. Weitere Regenerierungsverfahren sind u.a. in der WO 98/55228 und dem darin zitierten Stand der Technik beschrieben.

Um der allmählichen Deaktivierung von heterogenen Katalysatoren entgegenzuwirken, die bei der Umsetzung von organischen Verbindungen mit einem Hydroperoxid eingesetzt werden, ist es auch möglich, die Temperatur während der Umsetzung so zu regeln, daß die Deaktivierung kompensiert wird. Ebenso ist es denkbar, daß während der Umsetzung Temperatur und Druck geändert werden, um die Deaktivierung der Katalysatoren zu kompensieren. Dieses Verfahren ist beispielsweise in der WO 99/01445 beschrieben, wobei Temperatur und Druck während der Umsetzung von Propen mit einer aktiven Sauerstoff-Spezies an einem heterogenen Katalysator in flüssiger Phase gleichzeitig erhöht werden.

Eine weitere Möglichkeit, die Selektivität von beispielsweise Titansilikaliten zu beeinflussen, ist in J. Catal. 140 (1993) 71 - 83 beschrieben. Dort wird, ohne daß quantitative Ergebnisse angegeben wären, festgestellt, daß der Zusatz von Alkalimetallhydroxiden bei der Epoxidierung von niederen Olefinen in niedrigen Konzentrationen die Ausbeute erhöht, die Selektivität jedoch nicht beeinflußt. Bei höheren Basen-Konzentrationen jedoch wird die Aktivität des Titansilikalites gegebenenfalls vollständig unterdrückt. Die Epoxidierung von Allylchlorid, die in der gleichen Schrift beschrieben ist, wird durch den Zusatz von neutralen Salzen wie etwa LiCl bei Verwendung eines Titansilikaliten deutlich verschlechtert, während der Zusatz von HCl die Aktivität des Katalysators verbesserte. Der Zusatz des neutralen Salzes LiCl bei der Epoxidierung von 1-Buten störte dagegen die Aktivität des Katalysators nicht.

In der EP-A 0 712 852 wird offenbart, daß zur Verbesserung der Selektivität eines Titansilikalit-Katalysators, der zur Epoxidierung von olefinischen Verbindungen mittels Wasserstoffperoxid verwendet wird, ein nicht-basisches Salz eingesetzt wird. Die Versuche wurden dabei in Batch-Fahrweise bei konstanten Temperaturen durchgeführt.

Die EP-B 0 230 949 offenbart ein Verfahren zur Epoxidierung von olefinischen Verbindungen mittels Wasserstoffperoxid, in dem die verwendeten Katalysatoren, synthetische Zeolithe, in ihrer Selektivität dadurch verbessert werden, daß vor oder während der Reaktion Verbindungen zugegeben werden, die die Säuregruppen an der Katalysatoroberfläche neutralisieren. Das beschriebene Verfahren wurde unter isothermen Bedingungen durchgeführt.

Die EP-A 0 757 043 beschreibt ein Verfahren zur Herstellung von Epoxiden aus Olefinen und Wasserstoffperoxid in Gegenwart eines Titan-Atome enthaltenden Zeolithen als Katalysator, in dem dem Katalysator vor oder während der Reaktion neutral oder sauer reagierende Salze zugesetzt werden. Die Temperaturen bei der Umsetzung der Olefine mit Wasserstoffperoxid wurden dabei konstant gehalten.

In der Praxis zeigt sich, dass die Deaktivierung des Katalysators oft nicht gleichmäßig erfolgt. Vielmehr nimmt die Anfangsaktivität, die in der Regel sehr hoch ist, sehr schnell ab. Danach erfolgt eine relativ langsame Deaktivierung, die sich über mehrere hundert Stunden ausdehnen kann.

Eine Aufgabe der vorliegenden Erfindung war es, ein Verfahren bereitzustellen, das es erlaubt, flexibel auf diese unterschiedlich schnellen Deaktivierungsraten von heterogenen Katalysatoren zu reagieren, die bei Umsetzungen von organischen Verbindungen mit Hydroperoxiden auftreten.

Demgemäß betrifft die vorliegende Erfindung ein Verfahren zur Umsetzung einer organischen Verbindung mit einem Hydroperoxid unter Verwendung mindestens eines heterogenen Katalysators, das dadurch gekennzeichnet ist, dass während der Umsetzung sowohl der pH-Wert als auch die Temperatur des Reaktionsmediums geändert werden, wobei der heterogene Katalysator einen titanhaltigen Zeolithen umfaßt und die Änderung des pH-Wertes in einer pH-Wertemiedrigung und die Änderung der Temperatur in einer Temperaturerhöhung besteht.

Was die Verfahrensführung anbelangt, so sind sämtliche denkbaren Ausführungen möglich. Insbesondere kann die Umsetzung sowohl in Batch- als auch in kontinuierlicher Fahrweise durchgeführt werden. Selbstverständlich sind auch Mischformen denkbar, beispielsweise, wenn die Umsetzung in zwei oder mehr Stufen durchgeführt wird. Hierbei ist es denkbar, □ass in mindestens einer Stufe die Umsetzung in Batchfahrweise und in mindestens einer weiteren Stufe die Umsetzung in kontinuierlicher Fahrweise durchgeführt wird.

In einer bevorzugten Ausführungsform erfolgt die Umsetzung des Hydroperoxids mit der organischen Verbindung in koninuierlicher Fahrweise, wobei dem Reaktionsmedium, in dem die Umsetzung stattfindet, insbesondere ein kontinuierlicher Strom an Hydroperoxid zugeführt wird.

Demgemäß betrifft die vorliegende Erfindung auch ein Verfahren, wie oben beschrieben, das dadurch gekennzeichnet ist, daß dem Reaktionsmedium kontinuierlich eine Hydroperoxidlösung zugegeben wird.

Selbstverständlich ist es auch denkbar, dem Reaktionsmedium zwei oder mehr verschiedene Hydroperoxidlösungen zuzugeben, die sich beispielsweise in Hydroperoxidkonzentration, pH-Wert oder Temperatur unterscheiden können.

Der pH-Wert des Reaktionsmediums, der nach dem erfindungsgemäßen Verfahren während der Umsetzung des Hydroperoxids mit der organischen Verbindung verändert wird, kann über sämtliche denkbaren Wege erniedrigt werden.

So ist es beispielsweise denkbar, dem Reaktionsmedium direkt mindestens eine sauere oder mindestens eine basische Verbindung oder ein Gemisch aus zwei oder mehr davon zuzugeben. Die mindestens eine saure oder die mindestens eine basische Verbindung oder das Gemisch aus zwei oder mehr davon kann hierbei vor Zugabe zum Reaktionsmedium gegebenenfalls in einem geeigneten Lösungsmittel oder Lösungsmittelgemisch gelöst werden und die Lösung dem Reaktionsmedium zugegeben werden. Sowohl die Zugabe der sauren oder basischen Verbindung oder des Gemisches aus zwei oder mehr davon als auch die Zugabe der Lösung können kontinuierlich oder diskontinuierlich erfolgen, wobei es auch denkbar ist, daß beispielsweise zwei oder mehr gleiche oder verschiedene Verbindungen oder Gemische oder Lösungen separat voneinander zugegeben werden, wobei die Zugaben kontinuierlich und diskontinuierlich erfolgen können.

Der pH-Wert des Reaktionsmediums kann selbstverständlich auch über die Eduktströme, die im Verfahren kontinuierlich in das Reaktionsmedium einfließen, erniedrigt werden. So ist es denkbar, daß der pH-Wert eines Lösungsmittelstroms oder eines Eduktstroms, der die organische Verbindung enthält, oder auch eines Eduktstroms, der sowohl Lösungsmittel als auch organische Verbindung enthält, vor Zugabe zum Reaktionsmedium verändert wird und über diesen Weg der pH-Wert des Reaktionsmediums beeinflußt wird.

In einer bevorzugten Ausführungsform des erfindungsgemäßen Verfahrens wird der pH-Wert des Reaktionsmediums während der Umsetzung dadurch erniedrigt, daß der pH-Wert der Hydroperoxidlösung, die dem Reaktionsmedium kontinuierlich zugegeben wird, beeinflußt wird.

Daher betrifft die vorliegende Erfindung auch ein Verfahren, wie oben beschrieben, das dadurch gekennzeichnet ist, daß die Änderung des pH-Wertes des Reaktionsmediums durch Änderung des pH-Wertes der Hydroperoxidlösung, die dem Reaktionsmedium zugegeben wird, erreicht wird.

Die Steuerung des pH-Wertes durch den Hydroperoxidstrom kann dabei prinzipiell nach allen denkbaren Methoden erfolgen.

Beispielsweise ist es denkbar, einen Hydroperoxidstrom, der dem Reaktionsmedium zugegeben wird, vor der Zugabe in zwei oder mehr Teilströme aufzuteilen und den pH-Wert mindestens eines Teilstromes auf einen gewünschten Wert zu bringen. Im Anschluß daran können die mindestens zwei Teilströme wieder vereinigt werden und in einem Gesamtstrom dem Reaktionsmedium zugeführt werden. Ebenso ist es auch denkbar, die Teilströme separat voneinander oder, bei mehr als zwei Teilströmen, in geeigneten Unterkombinationen dem Reaktionsmedium zuzuführen. Die Einstellung des pH-Wertes des Reaktionsmediums kann dann entweder durch die Änderung des pH-Wertes mindestens eines Teilstroms oder durch geeignete Dosierung von Teilstrommengen oder durch eine Kombination dieser Methoden erreicht werden.

Ebenso ist es weiter denkbar, den pH-Wert des Reaktionsmediums durch eine Co-Dosierung von Hydroperoxidlösung und mindestens einer geeigneten basischen oder mindestens einer sauren Verbindung oder eines Gemisches aus zwei oder mehr davon oder, wie bereits oben beschrieben, gegebenenfalls einer Lösung mindestens einer dieser Verbindungen in einem geeigneten Lösungsmittel zu erniedrigen. Dabei können Hydroperoxidlösung und die mindestens eine saure oder basische Verbindung oder eine Lösung davon vor Zugabe zum Reaktionsmedium in geeigneten Anteilen zusammengeführt werden. Ebenso ist es denkbar, daß die Hydroperoxidlösung und die mindestens eine sauere oder basische Verbindung oder eine Lösung davon in separaten, geeignet dosierten Strömen in das Reaktionsmedium geleitet werden.

Die Umsetzung der organischen Verbindung mit Hydroperoxid kann prinzipiell in einer oder auch mehreren Stufen erfolgen. Insbesondere ist es denkbar, in einer ersten Stufe die organische Verbindung mit Hydroperoxid umzusetzen, aus dem durch die Umsetzung erhaltenen Gemisch nicht umgesetztes Hydroperoxid abzutrennen und in einer zweiten Stufe, die in einem anderen Reaktor durchgeführt wird als die erste Umsetzung, das abgetrennte Hydroperoxid erneut mit der organischen Verbindung umzusetzen.

Daher betrifft die vorliegende Erfindung auch ein Verfahren, wie oben beschrieben, das dadurch gekennzeichnet ist. daß es mindestens die folgenden Stufen (i) bis (iii) umfaßt:
(i) Umsetzung des Hydroperoxides mit der organischen Verbindung unter Erhalt einer Mischung, umfassend die umgesetzte organische Verbindung und nicht umgesetztes Hydroperoxid,
(ii) Abtrennung des nicht umgesetzten Hydroperoxides aus der aus Stufe (i) resultierenden Mischung,
(iii) Umsetzung des abgetrennten Hydroperoxides aus Stufe (ii) mit der organischen Verbindung,
wobei die Umsetzungen in den Stufen (i) und (iii) in mindestens zwei getrennten Reaktoren durchgeführt werden und die Änderung sowohl des pH-Wertes als auch der Temperatur des Reaktionsmediums in mindestens einem der Reaktoren, die in Stufe (i) und (iii) eingesetzt werden, durchgeführt wird.

Die Abtrennung des Hydroperoxids in der oben genannten Abtrennstufe (ii) kann im erfindungsgemäßen Verfahren nach allen gängigen Verfahren gemäß dem Stand der Technik durchgeführt werden. Sollten mehrere Abtrennstufen (ii) vorgesehen werden, können in unterschiedlichen Abtrennstufen auch unterschiedliche Abtrennmethoden eingesetzt werden.

Vorzugsweise erfolgt die Abtrennung des Hydroperoxides in der Abtrennstufe destillativ. Je nach den Anforderungen des Verfahrens ist dabei eine Abtrennung in einer oder mehreren Destillationskolonnen möglich. Vorzugsweise wird in der Abtrennstufe zur Abtrennung des Hydroperoxides eine Destillationskolonne verwendet.

Aus der Mischung, die aus der ersten Umsetzungsstufe (i), in der die organische Verbindung mit dem Hydroperoxid umgesetzt wird, resultiert, kann im erfindungsgemäßen Verfahren in einer Abtrennvorrichtung neben dem Hydroperoxid auch die umgesetzte organische Verbindung abgetrennt werden. Natürlich ist es auch möglich, nach Abtrennung des Hydroperoxids das verbleibende Reaktionsgut in eine weitere, speziell zu diesem Zweck vorgesehene Abtrennvorrichtung zu überführen und dort aus dem Reaktionsgut die umgesetzte organische Verbindung abzutrennen.

In beiden Fällen ist es beispielsweise möglich, die umgesetzte organische Verbindung in den Abtrennvorrichtungen zu sammeln und nach Beendigung der Umsetzungen der organischen Verbindung mit dem Hydroperoxid abzutrennen.

Bevorzugt wird die umgesetzte organische Verbindung jedoch in der jeweiligen Abtrennvorrichtung neben dem Hydroperoxid abgetrennt. Bei einer destillativen Abtrennung ist es beispielsweise möglich, die umgesetzte organische Verbindung über Kopf der Mischung zu entnehmen, und im Seitenabzug das Hydroperoxid aus der Mischung abzutrennen.

Im erfindungsgemäßen Verfahren ist es natürlich ebenfalls möglich, bei Verwendung einer Destillationsanlage als Abtrenneinrichtung das Hydroperoxid nicht über Seitenabzug, sondern über Sumpf aus der Mischung abzutrennen.

Erfolgt die Abtrennung des Hydroperoxids und/oder der umgesetzten organischen Verbindung in einer Destillationsanlage, ist es im erfindungsgemäßen Verfahren möglich, eventuell anfallende hochsiedende Komponenten der Mischung, die als Nebenprodukte aus der Umsetzung der organischen Verbindung mit dem Hydroperoxid anfallen, über Sumpf abzutrennen. Dabei ist es auch denkbar, beispielsweise durch Zugabe von vorzugsweise gasförmigen, niedrigsiedenden Komponenten, wie z.B. der organischen Verbindung, vorzugsweise Propen, an sich, die Sumpftemperatur zu erniedrigen.

Beispiele für solche niedrigsiedenden Komponenten sind u.a. Kohlenwasserstoffe mit 1 bis 4 Kohlenstoffatomen wie beispielsweise Methan, Ethan, Propan, Butan, Ethen oder Butene. Ebenso können beispielsweise Stickstoff oder Argon eingesetzt werden.

Selbstverständlich ist es im erfindungsgemäßen Verfahren möglich, auch mehrere organische Verbindungen mit dem Hydroperoxid umzusetzen.

Werden mehrere organische Verbindungen mit dem Hydroperoxid in Stufe (i) umgesetzt, so können in den Mischungen verschiedenartige Produkte, die aus den Umsetzungen resultieren, vorliegen. Werden diese wiederum in der Abtrennstufe (ü) destillativ abgetrennt, kann es notwendig sein, zur Abtrennung mehrere Destillationskolonnen vorzusehen.

Bevorzugt wird die Abtrennung in Stufe (ü) so geführt, daß eine flüssige Mischung, die das Hydroperoxid enthält, abgetrennt wird. Dabei ist es möglich, daß die abgetrennte Mischung, die das Hydroperoxid enthält, zusätzlich zum Hydroperoxid beispielsweise noch geringe Mengen an nicht umgesetzter organischer Verbindung und/oder umgesetzter organischer Verbindung enthält. Ebenso kann die Mischung, die das abgetrennte Hydroperoxid enthält, gegebenenfalls Lösungsmittel enthalten.

Wird in der Abtrennvorrichtung in Stufe (ü) auch die umgesetzte organische Verbindung abgetrennt, so resultiert aus dieser Abtrennung, aus der bevorzugt eine flüssige Mischung oder eine Flüssigkeit-Gas-Mischung erhalten wird, ein Strom, der neben der umgesetzten organischen Verbindung gegebenenfalls die nicht umgesetzte organische Verbindung und/oder geringe Mengen an Lösungsmittel enthält.

Nach dem Durchlauf der Stufen (i) und (ü) wird im erfindungsgemäßen Verfahren das abgetrennte Hydroperoxid in der Stufe (üi) in einem Reaktor, der von dem in Stufe (i) verwendeten Reaktor verschieden ist, erneut mit der organischen Verbindung umgesetzt.

Als Reaktoren können selbstverständlich alle denkbaren, für die jeweiligen Reaktionen am besten geeigneten Reaktoren eingesetzt werden. Dabei ist im erfindungsgemäßen Verfahren ein Reaktor nicht auf einen einzelnen Behälter beschränkt. Vielmehr ist es auch möglich, beispielsweise als Reaktor, der in Stufe (i) oder (iii) eingesetzt wird, eine Rührkesselkaskade einzusetzen.

Bevorzugt werden im erfindungsgemäßen Verfahren als Reaktoren Festbettreaktoren verwendet. Weiter bevorzugt werden als Festbettreaktoren Festbettrohrreaktoren eingesetzt.

Als Hydroperoxide können im erfindungsgemäßen Verfahren alle aus dem Stand der Technik bekannten Hydroperoxide, die für die Umsetzung der organischen Verbindung geeignet sind, verwendet werden.

Beispiele für solche Hydroperoxide sind etwa t-Butylhydroperoxid oder Ethylbenzolhydroperoxid, die ausgehend von Isobutan und Sauerstoff bzw. Ethylbenzol und Sauerstoff hergestellt werden können.

Bevorzugt wird als Hydroperoxidlösung im vorliegenden Verfahren eine Wasserstoffperoxidlösung, insbesondere eine wäßrige Wasserstoffperoxidlösung eingesetzt.

Zur Herstellung des Wasserstoffperoxids kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmann's Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Der pH-Wert der Hydroperoxidlösung, insbesondere der Wasserstoffperoxidlösung, kann prinzipiell nach allen gängigen Verfahren eingestellt werden. Dabei ist lediglich darauf zu achten, daß bei Zugabe von sauren oder basischen Verbindungen oder bei Zugabe einer Lösung, die saure oder basische Verbindungen umfaßt, zur Hydroperoxidlösung die nachfolgende Umsetzung der organischen Verbindung mit Hydroperoxid nicht nachteilig beeinflußt wird und der Stabilitätsbereich des eingesetzten Hydroperoxids nicht verlassen wird.

Demgemäß betrifft die vorliegende Erfindung auch ein Verfahren, wie oben beschrieben, das
dadurch gekennzeichnet ist, daß der pH-Wert der Hydroperoxidlösung
(a) durch Behandlung der Hydroperoxidlösung mit mindestens einem Ionentauscher oder
(b) durch Zugabe
   (aa) einer sauren Verbindung oder
   (bb) einer basischen Verbindung oder
   (cc) einer neutralen Verbindung oder
   (dd) eines Gemisches aus zwei oder mehr davon
   zur Hydroperoxidlösung oder
(c) durch eine Kombination der Methoden (a) und (b) geändert wird.

Hierbei sind prinzipiell sowohl stark basische als auch schwach basische Verbindungen oder sowohl stark saure als auch schwach saure Verbindungen geeignet. Insbesondere sind unter anderem die folgenden Salze denkbar:

Ammoniumsalze, Alkalisalze, wobei vor allem Lithium-, Natrium- und Kaliumsalze zu nennen sind, sowie Erdalkalisalze. Die Anionen dieser Salze umfassen beispielsweise Halogenide wie beispielsweise Chlorid und Bromid, Nitrat, Sulfat oder Hydroxid sowie die Anionen von Phosphor, Arsen, Antimon und Zinn enthaltenden Säuren wie z.B. Perchlorat, Phosphat, Hydrogenphosphat, Dihydrogenphosphat, Arsenat und Stannat. Auch andere Anionen wie beispielsweise Formiat, Acetat, Hydrogencarbonat oder Carbonat sind denkbar.

Als Beispiele seien unter anderem Lithiumchlorid, Lithiumbromid, Natriumbromid, Lithiumnitrat, Natriumnitrat, Kaliumnitrat, Lithiurnsulfat, Natriumsulfat, Kalitunsulfat, Natriumhydroxid, Kaliumhydroxid, Ammoniumhydroxid, Natriumcarbonat, Natriumhydrogencarbonat, Kaliumcarbonat, Lithiumcarbonat, Kaliumhydrogencarbonat, Lithiumhydrogencarbonat und Kaliumhydrogenphosphat sowie Lithium-, Magnesium-, Calcium-, Barium oder Ammoniumacetat. Ebenfalls zu nennen sind Carboxylate von Carbonsäuren, insbesondere von Carbonsäuren mit 1 bis 10 Kohlenstoffatomen, sowie Alkoholate von Alkoholen mit 1 bis 10 Kohlenstoffatomen. Weitere Beispiele sind unter anderem Ammoniumdihydrogenphosphat, Natriumdihydrogenphosphat, Kaliumdihydrogenphosphat, Dinatriumdihydrogenpyrophosphat, Tetranatriumpyrophosphat.

Als Lösungsmittel für die basischen oder saueren Verbindungen wird bevorzugt ein wäßriges Lösungsmittelgemisch eingesetzt, wobei Gemische mit dem bei der Umsetzung verwendeten Lösungsmittel, wie z. B. Methanol, bevorzugt sind.

Wie bereits oben beschrieben ist es auch möglich, den pH-Wert der Hydroperoxidlösung mittels Behandlung der Hydroperoxidlösung mit mindestens einem Ionentauscher zu erreichen. In dem erfindungsgemäßen Verfahren können prinzipiell Kationenaustauscher und Anionenaustauscher eingesetzt werden.

Wird nur ein Typ Ionenaustauscher verwendet, so ist der Einsatz mindestens eines Anionenaustauschers bevorzugt. Werden mehrere Ionenaustauscher verwendet, so können sie gleichzeitig oder nacheinander eingesetzt werden.

Im Rahmen der vorliegenden Erfindung lassen sich grundsätzlich alle dem Fachmann bekannten Ionenaustauscher einsetzen, beispielsweise organische Ionenaustauscher, etwa auf Polystyrolbasis, oder anorganische Ionenaustauscher, etwa Hydrotalcite sowie andere Schichtsilikate, die austauschbare Carbonat-, Hydrogencarbonat- oder Hydroxidgruppen enthalten können.

Beispiele für die im Rahmen der vorliegenden Erfindung besonders bevorzugten basischen Ionenaustauscher sind Polystyrolharze mit tertiären Amingruppen, etwa die kommerziell erhältlichen Anionenaustauscher Lewatit® MP62 und Lewatit® MP 63 sowie Dowex® MWA/1 und Dowex® AMW-500. Darüber hinaus ist auch die Verwendung von etwa quartäre Ammoniumgruppen enthaltenden Polystyrolharzen mit Hydroxid-Gegenionen denkbar. Beispielhaft seien hierbei die kommerziell erhältlichen Austauscher Lewatit® OC-1950 sowie Dowex® 1, Dowex® 2, Dowex® 11, Dowex® 21K und Dowex® 550A genannt.

Im Rahmen der bevorzugten Ausführungsform des erfindungsgemäßen Verfahrens, das die Stufen (i) und (iii) umfaßt, können sowohl der pH-Wert der Hydroperoxidlösung, mit der die organische Verbindung in Stufe (i) umgesetzt wird, als auch der pH-Wert der in Stufe (ii) abgetrennten Hydroperoxidlösung eingestellt werden. So können entweder der pH-Wert des Reaktionsmediums im Reaktor der Stufe (i) oder der pH-Wert des Reaktionsmediums im Reaktor der Stufe (iii) oder der pH-Wert beider Reaktionsmedien während der Umsetzung von Hydroperoxid mit der organischen Verbindung verändert werden.

Als heterogene Katalysatoren werden Katalysatoren eingesetzt, die als poröses oxidisches Material einen titanhaltigen Zeolithen umfassen.

Zeolithe sind bekanntermaßen kristalline Alumosilicate mit geordneten Kanal- und Käfigstrukturen, deren Porenöffnungen im Bereich von Mikroporen kleiner 0,9 nm liegen. Das Netzwerk solcher Zeolithe ist aufgebaut aus SiO₄⁻ und AlO₄⁻-Tetraedern, die über die gemeinsamen Sauerstoffbrücken verbunden sind. Eine Übersicht der bekannten Strukturen findet sich beispielsweise bei M. W. Meier, D. H. Olson, Ch. Baerlocher "Atlas of Zeolite Structure Types", 4. Auflage, Elsevier, London, 1996.

Zum Ausgleich der negativen Elektrovalenz, die durch den Einbau von Al(III) in das Si(IV)-Silicatgitter entsteht, findet man bei Zeolithen austauschfähige Kationen, insbesondere kann es sich dabei je nach Herstellverfahren um Kationen des Natriums, Kaliums, Lithiums oder Cäsiums handeln. Ersetzt man diese Kationen gegen Protonen, beispielsweise durch einen Ionenaustausch, so erhält man die entsprechenden aziden Festkörper mit Zeolithstruktur, die sogenannte H-Form.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur vom MFI-Typ, sowie Möglichkeiten zu ihrer Herstellung sind beschrieben beispielsweise in der EP-A 0 311 983 oder EP-A 0 405 978. Außer Silicium und Titan können solche Materialien auch zusätzliche Elemente wie z. B. Aluminium, Zirkonium, Zinn, Eisen, Kobalt, Nickel, Gallium, Bor oder geringe Menge an Fluor enthalten. In den im erfindungsgemäßen Verfahren verwendeten Zeolith-Katalysatoren kann das Titan des Zeoliths teilweise durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können.

Dabei sind im einzelnen Ti-Zeolithe des Strukturtyps ABW, ACO, AEI, AEL, AEN, AET, AFG, AFI, AFN, AFO, AFR, AFS, AFT, AFX, AFY, AHT, ANA, APC, APD, AST, ATN, ATO, ATS, ATT, ATV, AWO, AWW, BEA, BIK, BOG, BPH, BRE, CAN, CAS, CFI, CGF, CGS, CHA, CHI, CLO, CON, CZP, DAC, DDR, DFO, DFT, DOH, DON, EAB, EDI, EMT, EPI, ERI, ESV, EUO, FAU, FER, GIS, GME, GOO, HEU, IFR, ISV, ITE, JBW, KFI, LAU, LEV, LIO, LOS, LOV, LTA, LTL, LTN, MAZ, MEI, MEL, MEP, MER, MFI, MFS, MON, MOR, MSO, MTF, MTN, MTT, MTW, MWW, NAT, NES, NON, OFF, OSI, PAR, PAU, PHI, RHO, RON, RSN, RTE, RTH, RUT, SAO, SAT, SBE, SBS, SBT, SFF, SGT, SOD, STF, STI, STT, TER, THO, TON, TSC, VET, VFI, VNI, VSV, WEI, WEN, YUG, ZON sowie ITQ-4 oder einer Mischstruktur aus zwei oder mehr dieser Strukturen oder ein Gemisch aus zwei oder mehr davon zu nennen, wobei wiederum die mit MFI-Struktur, BEA-Struktur, MEL-Struktur, ITQ-4 bzw. MFI/MEL-Mischstruktur als besonders bevorzugt anzusehen sind. Zeolithe dieses Typs sind beispielsweise in der oben erwähnten Literaturstelle von W. M. Meier et al. beschrieben.

Als besonders bevorzugte Katalysatoren sind im einzelnen die Ti-enthaltenden Zeolith-Katalysatoren, die im allgemeinen als "TS-1", "TS-2", "TS-3", "ZSM-48" und "ZMS-12", jeweils mit Ti, TTM-1, Ti-RUT, titanhaltige Zeolithe des Typs "UTD-1", "CIT-5", "CTT-1" und "SSZ-24" sowie Ti-Zeclithe mit einer zu Zeolith-beta-isomorphen Gerüststruktur zu nennen.

Zum Beispiel werden Titanzeolithe eingesetzt, wie sie beispielsweise aus der US 3,329,481 bekannt sind. Bei derartigen Titanzeolithen wird ein Teil des ursprünglich im Silicatgitter vorhandenen Si(IV) durch Titan als Ti(IV) ersetzt. Weitere Titanzeolithe, insbesondere solche mit einer Kristallstruktur vom MFI-Typ sowie Möglichkeiten zu ihrer Herstellung sind u.a. in der US 4,410,501, EP-A 0 311 983, US 4,666,692, DE-A 30 47 798 oder in der BE 1 001 038 beschrieben. Weitere im Rahmen der vorliegenden Erfindung gut einsetzbare titanhaltige Zeolithe, die eine von der MFI-Struktur verschiedene Struktur aufweisen, sind beispielsweise in der EP-A 0 405 978 beschrieben. Außer Silicium und Titan können derartige Zeolithe auch zusätzliche Elemente wie Aluminium (beschrieben u.a. in der DE-A 3141 283), Gallium (EP-A 0 266 825), Bor (US 4,666,692) oder geringe Mengen an Fluor (EP-A 0 292 363) enthalten.

Weitere im Rahmen des Verfahrens der vorliegenden Erfindung verwendbare Zeolith-Katalysatoren sind u. a. in US-A 5,430,000 beschrieben.

Als weitere titanhaltige Zeolithe sind solche mit der Struktur des Ferrierits oder β-Zeoliths und des Mordenits zu nennen.

Ferner lassen sich im erfindungsgemäßen Verfahren folgende Zeolith-Katalysatoren verwenden:

Katalysatoren mit Zeolith-Struktur, wie sie in der DE-A 196 23 611.8 beschrieben sind.

Dabei handelt es sich um Oxidationskatalysatoren auf der Basis von Titansilikaten mit Zeolith-Struktur, wobei bzgl. der Zeolith-Struktur auf die vorstehend als bevorzugt angegebenen Strukturen verwiesen wird. Diese Katalysatoren sind dadurch gekennzeichnet, daß sie, wie in obiger Anmeldung detailliert beschrieben, durch verfestigende Formgebungsprozesse geformt werden.

Ferner können Oxidationskatalysatoren auf der Basis von Titansilikaten mit Zeolith-Struktur mit einem Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber, die ebenfalls dadurch gekennzeichnet sind, daß sie durch verfestigende Formgebungsprozesse geformt worden sind, verwendet werden. Derartige Katalysatoren sind in der DE-A 196 23 609.6 beschrieben.

Bzgl. der verfestigenden Formgebungsprozesse, der Bindemittel sowie der Hilfsmittel und der Struktur der Oxidationskatalysatoren wird auf die DE-A 196 23 611.8 Bezug genommen.

Der in der DE-A 196 23 609.6 beschriebene Oxidationskatalysator weist einen Gehalt von 0,01 bis 30 Gew.-%, insbesondere 0,05 bis 15 Gew.-%, vor allem 0,1 bis 8 Gew.-%, jeweils bezogen auf die Menge der Titan- oder Vanadium-Zeolithe, der genannten Edelmetallen auf. Hierbei wird Palladium besonders bevorzugt. Die Edelmetalle können auf den Katalysator in Form geeigneter Edelmetallkomponenten, beispielsweise in Form von wasserlöslichen Salzen, vor, während oder im Anschluß an den verfestigenden Formgebungsschritt aufgebracht werden.

Ferner können die folgenden Katalysatoren erfindungsgemäß verwendet werden:

Ein mindestens ein poröses oxidisches Material enthaltender Formkörper, der erhältlich ist durch ein Verfahren, das die folgenden Stufen umfaßt:
(I) Versetzen eines Gemischs enthaltend ein poröses oxidisches Material oder ein Gemisch aus zwei oder mehr davon mit einer Mischung enthaltend mindestens einen Alkohol und Wasser, und
(II) Kneten, Verformen, Trocknen und Calcinieren des gemäß Stufe (I) versetzten Gemischs.

Details bezüglich dieses Katalysators sind der DE-A 197 23 751.7 zu entnehmen.

Ferner können erfindungsgemäß Siliciumdioxid enthaltende Feststoffe verwendet werden, herstellbar durch ein Verfahren, das die folgende Stufe (I) umfaßt:
(I) Inkontaktbringen mindestens eines Vorläufers von Siliciumdioxid mit mindestens einem Strukturbildner in einem flüssigen Medium, dadurch gekennzeichnet, daß der Strukturbildner ein Polyethylenimin oder ein Gemisch aus zwei oder mehr davon ist.

Details bezüglich dieses Feststoffs sind der DE-A 197 32 865.2 zu entnehmen.

Weitere gut einsetzbare Katalysatoren stellen Formkörper dar, die einen inerten Träger und darauf aufgebracht mindestens ein Silicat, vorzugsweise ein kristallines Silicat, umfassen, erhältlich durch Aufbringen eines Gemischs enthaltend mindestens ein Silicat und mindestens einen Metallsäureester oder ein Hydrolysat davon oder eine Kombination aus Metallsäureester und Hydrolysat davon auf den inerten Träger, wie sie in der DE-A 197 54 924.1 beschrieben sind.

Ferner können erfindungsgemäß Fonnkörper verwendet werden, umfassend mindestens ein Silicat und mindestens ein Metalloxid, herstellbar durch ein Verfahren, das die folgende Stufe (i) umfaßt:
(i) Vermischen des mindestens einen Silicats mit mindestens einem Metalloxidsol, das einen niedrigen Gehalt an Alkali- und Erdalkalimetallionen aufweist,
wie sie in der DE-A 198 15 879.3 beschrieben sind.

Ferner lassen sich erfindungsgemäß Titansilicalite mit RUT-Struktur verwenden, herstellbar durch ein Verfahren, das die Schritte (i) und (ii) umfaßt:
(i) Herstellen einer Mischung aus mindestens einer SiO₂-Quelle und mindestens einer Titan-Quelle;
(ii) Kristallisation der Mischung aus (i) in einem Druckbehälter unter Zugabe mindestens einer Schablonenverbindung, wobei eine Suspension erhalten wird, dadurch gekennzeichnet, daß als Schablonenverbindung Amine oder Ammoniumsalze eingesetzt werden, die zur Stabilisierung von Käfigen der Silicatstruktur [4⁴5⁴6²] und [4⁴5⁶6⁵8¹] geeignet sind.

Details bezüglich dieser Katalysatoren lassen sich der DE-A 198 39 792.5 entnehmen.

Darüber hinaus lassen sich erfindungsgemäß die in der DE-A 198 47 630.2 beschriebenen Siliciumdioxide mit Meso- und Mikroporen verwenden, die vorzugsweise ein oder mehrere der folgenden Merkmale (i) bis (iii) aufweisen:
(i) Eine Summe der spezifischen Oberflächen der Meso- und Mikroporen von mindestens 300 m²/g;
(ii) eine Summe der Porenvolumen der Meso- und Mikroporen von mindestens 0,2 ml/g;
(iii) ein Maximum der Porendurchmesserverteilung der Mesoporen bei mindestens 3 nm.

Weitere Details bezüglich dieser Katalysatoren lassen sich der oben erwähnten Anmeldung entnehmen.

Weiter beschreibt die vorliegende Erfindung auch ein Verfahren, wie oben beschrieben, das dadurch gekennzeichnet ist, daß der titanhaltige Zeolith ein TS-1-Zeolith ist.

Unter den Reaktionen, die im erfindungsgemäßen Verfahren möglich sind, seien beispielhaft die folgenden genannt:
die Epoxidation von Olefinen wie z.B. die Herstellung von Propenoxid aus Propen und H₂O₂ oder aus Propen und Gemischen, die H₂O₂ in situ liefern;

Hydroxylierungen wie z.B. die Hydroxylierung mono-, bi- oder polycyclischer Aromaten zu mono-, di- oder höher-substituierten Hydroxyaromaten, beispielsweise die Umsetzung von Phenol und H₂O₂ oder von Phenol und Gemischen, die H₂O₂ in situ liefern, zu Hydrochinon;
die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
die Baeyer-Villiger-Oxidation.

Bevorzugt werden im erfindungsgemäßen Verfahren organische Verbindungen umgesetzt, die mindestens eine C-C-Doppelbindung aufweisen.

Als Beispiele für solche organischen Verbindungen mit mindestens einer C-C-Doppelbindung seien folgende Alkene genannt:
Ethen, Propen, 1-Buten, 2-Buten, Isobuten, Butadien, Pentene, Piperylen, Isopren, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen,
Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z. B. Acrylsäure, Methacrylsäure, Crotonsäure, Maleinsäure, Vinylessigsäure, ungesättigte Fettsäuren, wie z. B. Ölsäure, Linolsäure, Palmitinsäure, natürlich vorkommende Fette und Öle.

Bevorzugt werden im erfindungsgemäßen Verfahren Alkene verwendet, die 2 bis 8 Kohlenstoffatome enthalten. Besonders bevorzugt werden Ethen, Propen, und Buten umgesetzt. Insbesondere bevorzugt wird Propen umgesetzt.

Demgemäß beschreibt die vorliegende Erfindung auch ein Verfahren, das dadurch gekennzeichnet ist, daß die organische Verbindung Propen ist.

Was die Änderung der Temperatur des Reaktionsmediums, in dem die Umsetzung des Hydroperoxids mit der organischen Verbindung erfolgt, anbelangt, so sind im wesentlichen sämtliche denkbaren Verfahren möglich. Beispielsweise kann die Temperatur des Reaktionsmediums über die Temperatur mindestens eines Eduktstromes gesteuert werden, der dem Reaktionsmedium bevorzugt bei kontinuierlicher Verfahrensführung zugeführt wird.

Bevorzugt wird die Temperatur über eine geeignete Thermostatisierung des mindestens einen Reaktors variiert. Auch hierbei sind sämtlichen geeigneten Methoden anwendbar. Beispielsweise kann der mindestens eine Reaktor mit einem Doppelmantel versehen sein, durch den beispielsweise eine Flüssigkeit geleitet wird, über deren Temperatur die Temperatur des Reaktionsmediums im Reaktor eingestellt wird. Hierbei ist es selbstverständlich auch denkbar, verschiedene Zonen des mindestens einen Reaktors mit voneinander separierten Doppelmänteln zu versehen und so die verschiedenen Zonen mit beispielsweise Flüssigkeiten unterschiedlicher Temperatur zu umspülen. Die zonenweise unterschiedliche Temperierung ist selbstverständlich nicht auf Anordnungen beschränkt, bei denen der Reaktor mit einem mehreren Doppelmänteln versehen ist, sondern auch durch alle anderen geeigneten Methoden erreichbar.

Ebenso ist es denkbar, beispielsweise zwei oder mehr Hydroperoxidströme mit unterschiedlichen pH-Werten in verschiedene Zonen des mindestens einen Reaktors einzuleiten, um so beispielsweise zonenweise unterschiedliche pH-Werte im Reaktionsmedium einzustellen.

Natürlich ist es auch denkbar, verschiedene Zonen des mindestens einen Reaktors sowohl unterschiedlich zu temperieren als auch unterschiedliche pH-Werte des Reaktionsmediums in den Zonen einzustellen.

Im Rahmen der vorliegenden Erfindung ist es prinzipiell möglich, während der Umsetzung des Hydroperoxids mit der organischen Verbindung Temperatur und pH-Wert getrennt voneinander zu variieren. So ist es beispielsweise denkbar, in einem Schritt den pH-Wert, in einem nächsten Schritt die Temperatur und einem nächsten Schritt wiederum den pH-Wert zu variieren. Selbstverständlich ist es weiter denkbar, in einem Schritt den pH-Wert und in den beiden nächsten Schritten die Temperatur zu variieren. Ganz allgemein kann die Variation von pH-Wert und Temperatur in allen geeigneten und denkbaren Schritten erfolgen. Weiter ist es selbstverständlich möglich, pH-Wert und Temperatur gleichzeitig zu ändern. Vorzugsweise werden diese Parameter kontinuierlich, weiter bevorzugt gleichzeitig und kontinuierlich geändert.

Bevorzugt werden pH-Wert und Temperatur dergestalt geändert, daß eine konstante Aktivität des heterogenen Katalysators erreicht wird. Dies wird im allgemeinen dadurch erreicht, daß die Temperatur des Reaktionsmediums mit fortschreitender Versuchsdauer erhöht wird. Wird in einer bevorzugten Ausführungsform des erfindungsgemäßen Verfahrens ein titanhaltiger Silicalit als heterogener Katalysator eingesetzt, so wird der pH-Wert im Laufe der Umsetzung erniedrigt.

Dabei ist es selbstverständlich denkbar, daß im Laufe der Umsetzung auch eine oder mehrere Temperaturerniedrigungen oder eine oder mehrere pH-Wert-Erhöhungen vorgenommen werden, die beispielsweise nötig sind, um die Aktivität und Selektivität des Katalysators an einen Sollwert anzupassen

Ein besonderer Vorteil des erfindungsgemäßen Verfahrens ist darin zu sehen, daß bei geeigneter Variation des pH-Wertes des Reaktionsmediums wesentlich geringere Temperaturänderungen erforderlich sind, als dies ohne Änderung des pH-Wertes der Fall wäre.

Bei einem Reaktionsdruck von 30 bar liegen die Temperaturen, die im erfindungsgemäßen Verfahren eingestellt werden, im allgemeinen im Bereich von 0 bis 120 °C, bevorzugt im Bereich von 10 bis 90 °C und weiter bevorzugt im Bereich von 20 bis 70 °C. Der pH-Wert des Reaktionsmediums liegt im allgemeinen im Bereich von 2 bis 6 und besonders bevorzugt im Bereich von 3 bis 6. Im Dauerbetrieb werden die Temperatur um vorzugsweise 2°C/Tag oder weniger, weiter bevorzugt um 0,2 bis 1,0°C/Tag und der pH-Wert um vorzugsweise 0,5 Einheiten/Tag oder weniger, weiter bevorzugt um 0,01 bis 0,2 Einheiten/Tag geändert.

Neben den Parametern Temperatur und pH-Wert des Reaktionsmediums kann im Rahmen der vorliegenden Erfindung zusätzlich auch der Druck, unter dem die Umsetzung stattfindet, variiert werden. Hierbei ist es nicht ausgeschlossen, daß in einem oder mehreren Schritten Druck und pH-Wert bei konstanter Temperatur oder Druck und Temperatur bei konstantem pH-Wert variiert werden, solange, was die Gesamtdauer der Umsetzung anbelangt, pH-Wert und Temperatur des Reaktionsmediums variiert werden.

Was die Variation des Druckes anbelangt, so kann im erfindungsgemäßen Verfahren auf alle Methoden, die aus dem Stand der Technik bekannt sind oder in sonstiger Weise geeignet sind, zurückgegriffen werden. Bevorzugt wird allerdings bei Drücken gearbeitet, unter denen keine Gasphase vorliegt.

Natürlich kann im erfindungsgemäßen Verfahren der heterogene Katalysator vor Einsatz nach allen geeigneten Verfahren vorbehandelt werden. Eine Vorbehandlung von heterogenen Titansilikalit-Katalysatoren ist beispielsweise in der bereits oben erwähnten EP-B 0 230 949 beschrieben.

Ebenso ist es denkbar, den Katalysator nach der Umsetzung mittels sämtlicher geeigneter Verfahren zu regenerieren. Solche Regenerierungsverfahren sind beispielsweise in den bereits oben erwähnten J. Catal. 129 (1991) S. 159 - 166 und WO 98/55228 beschrieben.

In den folgenden Beispielen wird das erfindungsgemäße Verfahren näher erläutert.

### Beispiele

### Beispiel 1: Vergleichsbeispiel

In einem Rohrreaktor mit einem Durchmesser von 24 mm und einer Länge von 2000 mm, der mit einem Doppelmantel versehen war, wurden 565 g Katalysator eingefüllt. Der Katalysator war ein TS-1-Katalysator und wurde in Form von Formkörpern mit einem Durchmesser von 2 mm eingesetzt. Er wurde hergestellt gemäß der WO 97/31711.

Danach wurde der Reaktor mit Methanol geflutet, der Reaktionsdruck auf 30 bar eingestellt und die Methanoldosierung von 1560 g/h gestartet. Am Mantelraum des Reaktors wurde ein Thermostat zur Temperaturregelung angeschlossen und die Starttemperatur zunächst auf -5 °C eingestellt.

Folgende Edukte wurden dann mittels HPLC-Pumpen aus Druckvorlagen zudosiert:
- 134 g/h Propen, 99,5 %ig,
- 214 g/h Wasserstoffperoxid, wäßrig, 50 %ig, pH-Wert = 2.

Das austretende Produkt wurde entspannt und analysiert. Der Umsatz wurde durch Titration des nicht umgesetzten Wasserstoffperoxids mit Titanylsulfat bestimmt. Die Selektivität wurde mittels Gaschromatographie bestimmt. Im Laufe des Versuches wurde die am Thermostat eingestellte Temperatur so verändert, daß der Wasserstoffperoxid-Umsatz am Reaktorausgang konstant im Bereich von 85 ± 3 % lag. Die Propylenoxidselektivität bezüglich Wasserstoffperoxid blieb während des gesamten Versuchs weitgehend konstant und lag im Bereich von 92 bis 95 %.

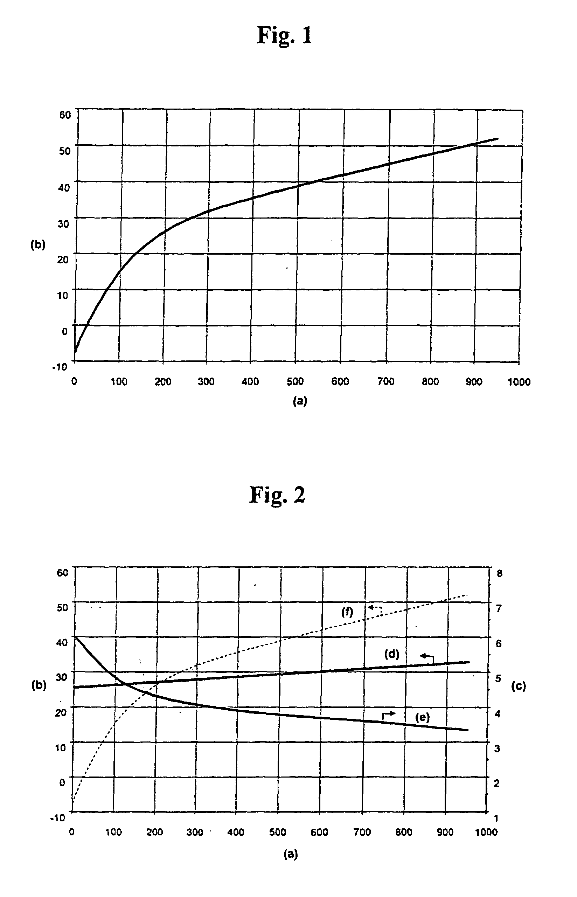

Der zeitliche Verlauf der Thermostattemperatur ist in Fig. 1 wiedergegeben.

Darin bezeichnen:
(a) Zeit/h
(b) Thermostattemperatur/°C

### Beispiel 2: Kontinuierliche Epoxidation von Propylen mit Temperatur- und pH-Anpassung mit dem Einsatz von Iouentauschern

Die Umsetzung wurde analog zu Beispiel 1 durchgeführt, wobei folgende Änderungen durchgeführt wurden: statt einer wurden zwei Dosierungen für Wasserstoffperoxidlösungen mit unterschiedlichen pH-Werten verwendet. Über die erste Dosiereinheit wurde eine wäßrige 50%ige Wasserstoffperoxidlösung mit einem pH-Wert von 6 zudosiert, über die zweite Dosiereinheit eine wäßrige 50%ige Wasserstoffperoxidlösung mit einem pH-Wert von 2 zudosiert. Die Wasserstoffperoxidlösung mit dem pH-Wert von 6 wurde durch Behandlung der kommerziellen Ware mit einem basischen Ionentauscher (z.B. Serdolit® Blue der Firma Boehringer Ingelheim oder Amberlite® IRA-68 der Firma Rohm & Haas) bei 0 °C hergestellt.

Die Ströme wurden mittels einer Glaselektrode pH-geregelt mit einer Verhältnisregelung so zudosiert, daß jeder gewünschte pH-Wert zwischen 2 und 6 eingestellt werden konnte.

Die auf den gewünschten pH-Wert eingestellte Wasserstoffperoxidlösung wurde dann wie im Beispiel 1 dem Reaktor zugeführt. Als Startbedingungen wurden eine Temperatur von 25 °C und ein pH-Wert von 6,0 eingestellt.

Das aus dem Reaktor austretende Produkt wurde dann entspannt und analysiert. Der Umsatz wurde durch Titration des nicht umgesetzten Wasserstoffperoxids mit Titanylsulfat bestimmt. Die Selektivität wurde mittels Gaschromatographie bestimmt. Im Laufe des Versuches wurde die am Thermostaten eingestellte Temperatur mit einer konstanten Rate von 0,2 °C/Tag erhöht und der pH-Wert der eingespeisten Wasserstoffperoxid-Lösung so abgesenkt, daß der Wasserstoffperoxid-Umsatz am Reaktorausgang konstant im Bereich von 85 ± 3 % lag. Die Propylenoxid-Selelctivität bezüglich Wasserstoffperoxid blieb während des gesamten Versuches konstant im Bereich von 92 bis 95 %.

Der zeitliche Verlauf der Thermostattemperatur und des pH-Wertes der eingesetzten Wasserstoffperoxid-Lösung sind in Fig. 2 wiedergegeben. Die Temperaturkurve aus Beispiel 1 ist als gestrichelte Kurve zum Vergleich eingezeichnet.

### In Fig. 2 bezeichnen:

(a) Zeit/h
(b) Thermostattemperatur/°C
(c) pH-Wert
(d) Temperaturkurve
(e) pH-Kurve
(f) Temperaturkurve aus Beispiel 1

Der Versuch wurde nach 950 h abgebrochen. Wie aus Fig. 2 ersichtlich ist, konnten durch die gleichzeitige Änderung von Temperatur und pH-Wert der benötigte Temperaturbereich und die benötigte Temperaturänderungsrate deutlich verringert werden.

## Patentansprüche

1. Verfahren zur Umsetzung einer organischen Verbindung mit einem Hydroperoxid unter Verwendung mindestens eines heterogenen Katalysators, **dadurch gekennzeichnet, dass** während der Umsetzung sowohl der pH-Wert als auch die Temperatur des Reaktionsmediums geändert werden, wobei der heterogene Katalysator einen titanhaltigen Zeolithen umfaßt und die Änderung des pH-Wertes in einer pH-Werteiniedrigung und die Änderung der Temperatur in einer Temperaturerhöhung besteht.

2. Verfahren nach Anspruch 1, **dadurch gekennzeichnet, daß** dem Reaktionsmedium kontinuierlich eine Hydroperoxidlösung zugegeben wird.

3. Verfahren nach Anspruch 2, **dadurch gekennzeichnet, daß** die Änderung des pH-Wertes des Reaktionsmediums durch Änderung des pH-Wertes der Hydroperoxidlösung, die dem Reaktionsmedium zugegeben wird, erreicht wird.

4. Verfahren nach einem der Ansprüche 1 bis 3, **dadurch gekennzeichnet, daß** es mindestens die folgenden Stufen (i) bis (iü) umfaßt:
(i) Umsetzung des Hydroperoxides mit der organischen Verbindung unter Erhalt einer Mischung, umfassend die umgesetzte organische Verbindung und nicht umgesetztes Hydroperoxid,
(ii) Abtrennung des nicht umgesetzten Hydroperoxides aus der aus Stufe (i) resultierenden Mischung,
(iii) Umsetzung des abgetrennten Hydroperoxides aus Stufe (ii) mit der organischen Verbindung,
wobei die Umsetzungen in den Stufen (i) und (iii) in mindestens zwei getrennten Reaktoren durchgeführt werden und die Änderung sowohl des pH-Wertes als auch der Temperatur des Reaktionsmediums in mindestens einem der Reaktoren, die in Stufe (i) und (iii) eingesetzt werden, durchgeführt wird.

5. Verfahren nach einem der Ansprüche 2 bis 4, **dadurch gekennzeichnet, daß** der pH-Wert der Hydroperoxidlösung
(a) durch Behandlung der Hydroperoxidlösung mit mindestens einem Ionentauscher oder
(b) durch Zugabe
(aa) eines sauren Salzes oder
(bb) eines basischen Salzes oder
(cc) einer neutralen Verbindung oder
(dd) eines Gemisches davon
zur Hydroperoxidlösung oder
(c) durch eine Kombination der Methoden (a) und (b) geändert wird.

6. Verfahren nach einem der Ansprüche 2 bis 5, **dadurch gekennzeichnet, daß** die Hydroperoxidlösung eine wäßrige Wasserstoffperoxidlösung ist.

7. Verfahren nach einem der Ansprüche 1 bis 6, **dadurch gekennzeichnet, daß** die organische Verbindung mindestens eine C-C-Doppelbindung aufweist.

8. Verfahren nach einem der Ansprüche 1 bis 7, **dadurch gekennzeichnet, daß** zusätzlich zu Temperatur und pH-Wert des Reaktionsmediums der Druck, unter dem die Umsetzung durchgeführt wird, geändert wird.

9. Verfahren nach einem der Ansprüche 6 bis 8, **dadurch gekennzeichnet, dass** bei einem Reaktionsdruck von 30 bar die Temperatur im Bereich von 0 bis 120 °C und der pH-Wert im Bereich von 2 bis 6 liegt.

10. Verfahren nach Anspruch 9, **dadurch gekennzeichnet, dass** die Temperatur um 2 °C/Tag oder weniger und der pH-Wert um 0,5 Einheiten/Tag oder weniger geändert wird.

## Claims

1. A process for reacting an organic compound with a hydroperoxide using at least one heterogeneous catalyst, wherein both the pH and the temperature of the reaction medium are changed during the reaction, with the heterogeneous catalyst comprising a titanium-containing zeolite and the change in the pH being a pH decrease and the change in the temperature being a temperature increase.

2. A process as claimed in claim 1, wherein a hydroperoxide solution is added continuously to the reaction medium.

3. A process as claimed in claim 2, wherein the change in the pH of the reaction medium is achieved by changing the pH of the hydroperoxide solution which is added to the reaction medium.

4. A process as claimed in any of claims 1 to 3, which comprises at least the steps (i) to (iii):
(i) reacting the hydroperoxide with the organic compound to give a mixture comprising the reacted organic compound and unreacted hydroperoxide,
(ii) separating the unreacted hydroperoxide from the mixture resulting from step (i),
(iii) reacting the hydroperoxide which has been separated off in step (ii) with the organic compound,
where the reactions in steps (i) and (iii) are carried out in at least two separate reactors and both the pH and the temperature of the reaction medium are changed in at least one of the reactors used in steps (i) and (iii).

5. A process as claimed in any of claims 2 to 4, wherein the pH of the hydroperoxide solution is changed
(a) by treatment of the hydroperoxide solution with at least one ion exchanger or
(b) by addition of
(aa) an acidic salt or
(bb) a basic salt or
(cc) a neutral compound or
(dd) a mixture or two or more thereof
to the hydroperoxide solution or
(c) by a combination of methods (a) and (b).

6. A process as claimed in any of claims 2 to 5, wherein the hydroperoxide solution is an aqueous hydrogen peroxide solution.

7. A process as claimed in any of claims 1 to 6, wherein the organic compound contains at least one C-C double bond.

8. A process as claimed in any of claims 1 to 7, wherein not only the temperature and pH of the reaction medium but also the pressure under which the reaction is carried out is changed.

9. A process as claimed in any of claims 6 to 8, wherein at a reaction pressure of 30 bar the temperature is in the range from 0 to 120°C and the pH is in the range from 2 to 6.

10. A process as claimed in claim 9, wherein the temperature is changed by 2°C/day or less and the pH is changed by 0.5 unit/day or less.

## Revendications

1. Procédé pour faire réagir un composé organique avec un hydroperoxyde, avec utilisation d'au moins un catalyseur hétérogène, **caractérisé en ce que**, pendant la réaction, non seulement la valeur du pH mais aussi la température du milieu réactionnel sont modifiées, le catalyseur hétérogène comportant une zéolite contenant du titane et la modification de la valeur du pH consistant en un abaissement de la valeur du pH et la modification de la température en une augmentation de la température.

2. Procédé suivant la revendication 1, **caractérisé en ce qu'**au milieu réactionnel on ajoute en continu une solution d'hydroperoxyde.

3. Procédé suivant la revendication 2, **caractérisé en ce que** la modification de la valeur de pH du milieu réactionnel est obtenue par modification de la valeur du pH de la solution d'hydroperoxyde qui est ajoutée au milieu réactionnel.

4. Procédé suivant l'une des revendications 1 à 3, **caractérisé en ce qu'**il comporte au moins les étapes suivantes (i) à (iii) :
(i) une réaction de l'hydroperoxyde avec le composé organique, avec obtention d'un mélange comportant le composé organique ayant réagi et l'hydroperoxyde qui n'a pas réagi,
(ii) une séparation de l'hydroperoxyde qui n'a pas réagi à partir du mélange résultant de l'étape (i),
(iii)une réaction de l'hydroperoxyde séparé de l'étape (ii) avec le composé organique,
procédé dans lequel les réactions dans les étapes (i) et (iii) sont effectuées dans au moins deux réacteurs séparés, la modification non seulement de la valeur du pH mais aussi de la température du milieu réactionnel étant effectuée dans au moins un des réacteurs qui sont mis en oeuvre dans l'étape (i) et dans l'étape (iii).

5. Procédé suivant l'une des revendications 2 à 4, **caractérisé en ce que** la valeur du pH de la solution d'hydroperoxyde est modifiée
(a) par traitement de la solution d'hydroperoxyde avec au moins un échangeur d'ions, ou
(b) par addition
(aa) d'un sel acide, ou
(bb) d'un sel basique, ou
(cc) d'un composé neutre, ou
(dd) d'un mélange de ceux-ci
à la solution d'hydroperoxyde, ou
(c) par une combinaison des méthodes (a) et (b).

6. Procédé suivant l'une des revendications 2 à 5, **caractérisé en ce que** la solution d'hydroperoxyde est une solution aqueuse de peroxyde d'hydrogène.

7. Procédé suivant l'une des revendications 1 à 6, **caractérisé en ce que** le composé organique présente au moins une double liaison C-C.

8. Procédé suivant l'une des revendications 1 à 7, **caractérisé en ce que**, en plus de la température et de la valeur du pH du milieu réactionnel, on modifie la pression sous laquelle la réaction est effectuée.

9. Procédé suivant l'une des revendications 6 à 8, **caractérisé en ce que**, à une pression réactionnelle de 30 bars, la température est de l'ordre de 0 à 120°C et la valeur du pH de l'ordre de 2 à 6.

10. Procédé suivant la revendication 9, **caractérisé en ce que** la température est modifiée de 2°C/jour ou moins et la valeur du pH de 0,5 unité/jour ou moins.
